# EUROPÄISCHE PATENTSCHRIFT

(11) **EP 0 329 948 B1**
(45) Veröffentlichungstag und Bekanntmachung des Hinweises auf die Patenterteilung: **26.05.1993**
(21) Anmeldenummer: 89101036.5
(22) Anmeldetag: 21.01.1989
(51) Int. Cl.: A61K 31/22, A61K 31/135, A61K 31/165, A61K 31/40, A61K 31/275, A61K 31/17, A61K 31/44, A61K 31/535, A61K 31/425

(54) **Verwendung von Betablockern zur Behandlung der Progression der kindlichen Achsenmyopie**
Use of beta-adrenergic blocking agents in the treatment of the progression of infantile myopia
Application des bêta-bloquants pour le traitement de la progression de la myopie infantile

(30) Priorität: 25.02.1988 DE 3805882
(43) Veröffentlichungstag der Anmeldung: 30.08.1989
(73) Patentinhaber: Dr. Gerhard Mann chem.-pharm. Fabrik GmbH, D-13578 Berlin (DE)
(72) Erfinder: Tiburtius, Heinfried, Prof. Dr. med., D-1000 Berlin 33 (DE)
(74) Vertreter: Siewers, Gescha, Dr.

(56) Entgegenhaltungen:
- Doc. Ophthal. Proc. Series, Band 28, 1981, S. 249-253, Dr. W. Junk Publ., The Hague, NL; T, Stuart-Black Kelly
- Acta Ophtalmol., Band 65, Suppl. 132, 1987, S. 132; H. Jensen et al.
- Acta Ophtalmol., Band 66, Suppl. 185, 1988, S. 130-131; A. Hosaka
- Acta Ophtalmol., Band 59, Nr. 5, 1981, S. 759-762; E. Goldschmidt
- Klin. MBL. Augenheilk., Band 182, Nr. 2, 1983, S. 153-155; N. Demmler
- Münchener Medizinische Wochenschrift, Band 132, Nr. 24, 1990, S. 17

## Beschreibung

Die Erfindung betrifft die Verwendung von Betablockern zur Herstellung eines Arzneimittels zur Behandlung der Progression der kindlichen Achsenmyopie.

Myopie oder Kurzsichtigkeit ist eine Erkrankung des Auges, bei der die parallel einfallenden Lichtstrahlen vor der Netzhaut vereinigt werden, wobei diese Erkrankung zwei völlig unterschiedliche Gründe haben kann, nämlich entweder, daß die Brechung der Strahlen im Auge zu stark ist, wie es gelegentlich bei Diabetikern sowie bei Beginn bestimmter Formen des grauen Stars auftritt und wobei es sich dann um eine sogenannte Brechungsmyopie handelt, oder dadurch, daß der Augapfel zu lang ist, also eine sogenannte Achsenmyopie vorliegt, die zum Teil erblich ist. Einzige bislang bekannte Therapie bei der Achsenmyopie ist die Benutzung von Konkavgläsern, wodurch der Fokus wieder auf die Netzhaut zurückverlegt wird oder von Contaktschalen; wodurch mechanisch durch Druck auf das Auge die Längenzunahme gebremst werden soll.

Die kindliche Achsenmyopie entwickelt sich in den meisten Fällen in der Schulzeit etwa zwischen dem 10. und 12. Lebensjahr und nur selten früher und nimmt in der Regel bis etwa zum 17. Lebensjahr zu. Die Ursache für die Erkrankung ist nicht genau bekannt, obgleich eine Zunahme der Kurzsichtigkeit festzustellen ist. Mit Beendigung der Wachstumsperiode bei Kindern, d.h. etwa um das 17. Lebensjahr herum, haben sich die bis dahin noch weichen Bulbushüllen soweit verfestigt, daß eine weitere Längenzunahme des Augapfels, was gleichbedeutend ist mit einer Zunahme der Kurzsichtigkeit, in der Regel nicht mehr zu erwarten ist.

Es gibt aber auch relativ seltene Fälle angeborenen Glaukoms, bei dem bereits im ersten oder zweiten Lebensjahr der Augendruck stark erhöht ist. Wenn es nicht gelingt, diesen Druck zu normalisieren, bläht er den Augapfel ballonartig auf. Das erkrankte Kind wird hochgradig kurzsichtig und kann schließlich infolge Zerstörung der Sehnerven erblinden. Im Gegensatz dazu steht die Tatsache, daß bei einer Augeninnendrucksteigerung, also einem Glaukom, bei Erwachsenen keine Kurzsichtigkeit provoziert wird, da das Glaukom kaum vor dem 40. Lebensjahr in Erscheinung tritt und dann die Bulbushüllen so fest sind, daß sich keine Kurzsichtigkeit mehr entwickelt.

Die heutige Standardtherapie zur Behandlung des Glaukoms besteht in der Verabreichung von Betablockern, die ursprünglich in der Herz- bzw. Kreislauftherapie angewendet wurden. Betablocker sind nicht nur in der Lage, den erhöhten Blutdruck zu senken, sondern auch durch Hemmung der Kammerwasserproduktion den erhöhten Augeninnendruck zu normalisieren. Die Verwendung von Betablockern bei der glaukombedingten Myopie wird beispielsweise in DAZ, 124, Nr. 16, 19.04.1984, S. 281-287 beschrieben. In der Regel werden Betablocker als Isomerengemisch eingesetzt, aber in den letzten Jahren hat sich herausgestellt, daß in manchen Fällen die Antipoden unterschiedlich wirksam sind; meist sind die linksdrehenden Antipoden stärker beta-sympatholytisch aktiv.

Es ist bekannt, daß der Augeninnendruck, bestimmt durch Kammerwasserproduktion und Abfluß, bei Kindern nicht pathologisch erhöht ist, sondern im normalen Bereich zwischen etwa 10 und 20 mm/Hg schwankt. Völlig überraschend wurde jetzt festgestellt, daß Betablocker in der Lage sind, die Entwicklung und Progression der kindlichen Achsenmyopie zu verhindern, wenn sie regelmäßig in relativ kleinen Dosen appliziert werden. Eine Erklärung für dieses Phänomen läßt sich bisher nicht sicher geben, da, wie ausgeführt, Kinder, die eine Achsenmyopie entwickeln, regelmäßig über einen normalen Augeninnendruck verfügen.

Zu den anwendbaren Betablockern gehören die folgenden Betablocker, die bislang auch bei der Glaukombehandlung zum Einsatz gekommen sind: Methypranol, (oder Metipranolol) Propranolol, Timolol, Bupranolol und Penbutolol. Die Substanzen können als Isomerengemisch oder auch jeweils in der links- oder rechtsdrehenden Form eingesetzt werden.

Die Dosierung beträgt in der Regel 2 x täglich ein Tropfen in jedes Auge bei einer Dosierung des Betablockers von 0,1 %. Diese Therapie muß von Beginn oder vor Beginn der zu erwartenden Kurzsichtigkeit an regelmäßig bis Ende der Wachstumsperiode des Kindes, also in der Regel bis zum 17. Lebensjahr durchgeführt werden.

Im folgenden wird die Erfindung anhand von Beispielen näher erläutert:

### Beispiel 1

### Herstellung der Betablockerlösung.

In 97,25 kg Wasser für Injektionszwecke werden 0,355 kg 1 N-Salzsäure gelöst und darin anschließend unter kräftigem Rühren 0,100 kg Metipranolol zugegeben. In die klare Lösung werden nacheinander 0,010 kg Benzalkoniumchlorid, 0,500 kg Natriumchlorid, 0,010 Natriumeditat, 1,000 kg Glycerol 85%-ig und 1,800 kg PVP (mittleres Molekulargewicht 90.000) eingegeben und unter Rühren gelöst. Nachdem eine klare Lösung entstanden ist, wird der pH-Wert kontrolliert und eventuell mit 1 N-Natronlauge auf pH 5,5 eingestellt.

Die Lösung wird über ein dampfsterilisiertes 0,2 µm-Filter unter Verwendung von sterilfiltriertem Stickstoff als Druckgas in eine dampfsterilisierte Vorlage filtriert. Die Lösung wird dann unter Laminar-Flow in sterile ml-Behältnisse aus Hochdruckpolyethylen unter Verwendung steriler Verschlußkappen automatisch abgefüllt.

Die Konzentration des Wirkstoffes beträgt 0,1 %.

In entsprechender Weise können auch Lösungen der übrigen an und für sich bekannten Betablocker hergestellt werden. Der pH der Lösungen sollte auf etwa 5,5 bis 6 eingestellt werden und die Lösungen sollten durch Zugabe von Natriumchlorid isotonisch gehalten werden.

### Beispiel 2

Die Wirksamkeit der Verwendung von Betablockern zur Behandlung der kindlichen Myopie wurde in Gruppen von 15 Kindern klinisch untersucht. Die Kinder zeigten vorher kontinuierliche Zunahme der Kurzsichtigkeit. Zur Behandlung erhielten die Kinder 2 x täglich in jedes Auge einen Tropfen einer 0,1%-igen wässrigen Lösung von Metipranolol. Nebenwirkungen systemischer Art wurden nicht beobachtet. Nach jeweils 2 Monaten wurden die üblichen Untersuchungen zur Bestimmung der Sehkraft durchgeführt. Bei keinem der Probanten wurde eine Progredienz der Myopie festgestellt. Die bisherigen klinischen Untersuchungen erstrecken sich über einen mehrjährigen Zeitraum, währenddessen keine Verschlechterung der Sehschärfe zu beobachten war.

## Patentansprüche

1. Verwendung von Metipranolol, Bupranolol, Timolol, Penbutolol oder Propranolol zur Herstellung von 0,1 %-igen Lösungen zur topischen Anwendung am Auge zur Verhinderung oder Behandlung der progredienten kindlichen Achsenmyopie bei normalem Augeninnendruck.

2. Verwendung von Metipranolol zur Herstellung von 0,1 %-igen lösungen zur topischen Anwendung am Auge zur Verhinderung oder Behandlung der progredienten kindlichen Achsenmyopie bei normalem Augeninnendruck.

## Claims

1. Use of Metipranolol, Bupranolol, Timolol, Penbutolol, or Propranolol for the manufacture of 0,1 % solutions for the topical application to the eye, for the prevention or treatment of progredient infantile axial myopia in the case of normal intraocular pressure.

2. Use of Metipranolol for the manufacture of 0,1 % solutions for the topical application to the eye, for the prevention or treatment of progredient infantile axial myopia in the case of normal intraocular pressure.

## Revendications

1. Utilisation de metipranolol, bupranolol, timolol, penbutolol ou propanolol pour la manufacture des solutions à 0.1 pour-cent (%) pour l'application topique aux yeux pour le prévention ou le traitement de progressive infantile myopie axiale en cas de pression intraoculaire normale.

2. Utilisation de metipranolol pour la manufacture des solutions à 0.1 pour-cent (%) pour l'application topique aux yeux, pour le prévention ou le traitement de progressive infantile myopie axiale en cas de pression intraoculaire normale.
